(19)

Europäisches
Patentamt
European
Patent Office
Office européen
des brevets

(11) **EP 4 537 932 A1**

(12) **EUROPEAN PATENT APPLICATION**
published in accordance with Art. 153(4) EPC

(43) Date of publication:
**16.04.2025 Bulletin 2025/16**

(21) Application number: **23920161.9**

(22) Date of filing: **21.12.2023**

(51) International Patent Classification (IPC):
**B01J 20/26** (2006.01)   **B01J 20/28** (2006.01)
**B01J 20/30** (2006.01)   **C07K 1/22** (2006.01)

(52) Cooperative Patent Classification (CPC):
**B01J 20/26; B01J 20/28; B01J 20/30; C07K 1/22**

(86) International application number:
**PCT/KR2023/021323**

(87) International publication number:
**WO 2024/162612 (08.08.2024 Gazette 2024/32)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority:   **01.02.2023   KR 20230013569
11.05.2023   KR 20230061331**

(71) Applicant: **LG Chem, Ltd.**
**Yeongdeungpo-gu**
**Seoul 07336 (KR)**

(72) Inventors:
• **KIM, Yeji**
  **Daejeon 34122 (KR)**
• **KIM, Minchae**
  **Daejeon 34122 (KR)**
• **OH, Seungjun**
  **Daejeon 34122 (KR)**
• **KIM, Jee Seon**
  **Daejeon 34122 (KR)**
• **KIM, Yunseop**
  **Daejeon 34122 (KR)**

(74) Representative: **Plasseraud IP**
**104 Rue de Richelieu**
**CS92104**
**75080 Paris Cedex 02 (FR)**

(54)   **POROUS PARTICLE AND METHOD FOR MANUFACTURING SAME**

(57)   The present application relates to porous particles and a method for preparing the same. The porous particles provided according to the present application have characteristics suitable for protein purification, especially affinity chromatography.

【FIG. 1】

**Description**

**[TECHNICAL FIELD]**

CROSS-REFERENCE TO RELATED APPLICATION(S)

This application claims the benefit of Korean Patent Application No. 10-2023-0013569 filed on February 1, 2023 and Korean Patent Application No. 10-2023-0061331 filed on May 11, 2023 in the Korean Intellectual Property Office, the disclosures of which are incorporated herein by reference in their entirety.

The present application relates to porous particles and a method for preparing the same. More specifically, the present application relates to porous particles for affinity chromatography and a method for preparing the same.

**[BACKGROUND ART]**

As the field of biopharmaceuticals and regenerative medicine expands, the demand for systems that can efficiently separate and purify cells, tissues, microorganisms, and the like is increasing.

In conventional affinity chromatography techniques, purification resins made from hydrophilic agarose have been mainly used, but such resins are swollen by a buffer solution during the purification process and thus cause reduction in strength, which results in a deterioration of the purification speed and purification efficiency. Attempts have therefore been made to prepare synthetic polymers with improved strength.

When using purification resins, non-specific binding of impurities occurs mainly through hydrophobic interaction. The adsorbed impurities may foul the purified resin particles and have adverse effects on the activity of Protein A ligand immobilized onto the resin and the binding of the target material. Further, if impurities elute together with the purification target material, the purity of the final product decreases, and if impurities are administered to patients, they may trigger an immune response.

Therefore, there is a need to develop a technique regarding particles that can reduce non-specific adsorption, and a method for preparing the same.

**[DETAILED DESCRIPTION OF THE INVENTION]**

**[Technical Problem]**

It is an object of the present application to provide porous particles having an open pore system.

It is another object of the present application to provide porous particles which is reduced in non-specific adsorption.

It is another object of the present application to provide particles (e.g., column resin) for protein purification.

It is yet another object of the present application to provide porous particles suitable for affinity chromatography.

It is a further object of the present application to provide a method for preparing porous particles as described above.

The above and other objects of the present application can be all solved by the present application which is described in detail below.

**[Technical Solution]**

According to an embodiment of the present application, porous particles and a method for preparing the same are provided. The particles can be used as particles for protein purification (column resin), specifically for affinity chromatography. Herein, "porous particles" may mean particles having pores in each inside and/or on each surface of the particles. More specifically, the porous particles may mean particles having an open pore system.

Particles for protein purification (column resin) are exposed to solutions containing antibody proteins and other cell culture-derived impurities. Therefore, a situation occurs in which impurities are non-specifically adsorbed on the particle surface. In order to ensure binding between particles and target materials and increase the purity of the final product, non-specific adsorption of impurities must be lowered. The inventors of the present application have experimentally confirmed that the preparation method described below and the particles prepared thereby can reduce such a non-specific adsorption, and completed the invention of the present application.

Specifically, in order for the porous particles to have sufficient purification capacity in affinity chromatography, the target material (e.g., IgG) must be able to bind to the protein A ligand as much as possible. In order to provide such binding as much as possible, it is necessary to immobilize a large amount of the ligand to the particles because the specific surface area of the particles is high. In order to increase the specific surface area, the pores of the particles must be sufficiently large, and the number of pores (or porosity) must also be ensured at a high level. This is because, if the pores of the particles are small, the ligand may not be able to move into the inside of the particles when immobilizing the ligand, and the target material may not be able to move in the inside when purifying the target material. In addition, in order for smooth purification to proceed, the shape of the particles needs to be uniform.

Further, during the purification process, adsorption between impurity proteins and purified particles mainly occurs through hydrophobic bonds between impurity proteins and purified particles, so that as the particles have more hydrophilic groups, the hydrophobic bonds (non-specific adsorption) may be reduced.

In consideration of such points, the inventors of the present application conducted extensive research on the materials used for preparing porous particles, their content, reaction conditions, surface characteristics of the particles, and the like, and completed the invention of the present application.

Now, the invention of this application will be described in more detail.

In one embodiment related to the present application, this application relates to a method for preparing porous particles. The porous particles prepared according to the method of the present application may have characteristics suitable for protein purification purposes, specifically affinity chromatography.

Specifically, the method comprises mixing a dispersed phase composition(A) and a continuous phase composition(B) in a reactor and then subjecting the mixture to a suspension polymerization reaction. Wherein, "dispersed phase composition" means a composition that can form a dispersed phase (or droplet) after being mixed with a continuous phase composition, and "continuous phase composition" means a composition that can form a continuous phase after being mixed with a dispersed phase composition.

In an embodiment of the present application, the dispersed phase composition includes an epoxy group-containing polymerizable monomer(b1), a crosslinking agent(b2), and a porogen(b3). In an embodiment of the present application, the dispersed phase composition may further include an initiator(b4) in addition to the epoxy group-containing polymerizable monomer(b1), the crosslinking agent(b2), and the porogen (b3). In another embodiment, the dispersed phase composition may be a mixture of the above-mentioned components.

By suspension polymerization of the dispersed phase composition, a suspension polymerization product containing a unit derived from the polymerizable monomer(b1) and the crosslinking agent(b2) can be obtained. More specifically, a suspension polymerization product which includes a unit derived from the polymerizable monomer(b1) and the crosslinking agent(b2), or is a reaction product between a polymerizable monomer(b1) (progressed by an initiator) and the crosslinking agent(b2), can be obtained by the suspension polymerization. The suspension polymerization product may be particles, or a particle group including the same, and each individual particle may have particle characteristics described below.

In one exemplary embodiment, the polymerizable monomer(b1) may be a monomer containing an unsaturated bond between carbons and an epoxy group (or glycidyl group). The type of such polymerizable monomer is not particularly limited, but may be, for example, a (meth)acrylate monomer having an epoxy group, and more specifically, glycidyl (meth) acrylate, 4,5-epoxybutyl (meth)acrylate, or 9,10-epoxy stearyl (meth)acrylate may be mentioned. However, the types of polymerizable monomers included in the dispersed phase composition of the present application are not specified by the monomers listed above.

The dispersed phase composition further comprises a crosslinking agent(b2). In an embodiment of the present application, a compound containing at least two double bonds between carbons (e.g., a vinyl group) that can participate in a crosslinking reaction may be used as the crosslinking agent.

The crosslinking agents that can be used according to embodiments of the present application include, for example, polyfunctional (meth)acrylates, such as ethylene glycol di(meth)acrylate, trimethylolpropane tri(meth)acrylate, pentaerythritol tri(meth)acrylate, dipentaerythritol hexa(meth)acrylate, dipentaerythritol hexamethacrylate or trimethylpropane trimethacrylate. Alternatively, one or more of the crosslinking agents listed above may be used in combination.

In one exemplary embodiment, the crosslinking agent compound may be one that does not contain a hydrophilic group (e.g., a hydroxy group). As a result of experimental confirmation, if the crosslinking agent contains a hydrophilic group in addition to the double bond between carbons, the reaction rate of the crosslinking agent may decrease, which may cause conditions that make it difficult to form sufficient particle pores.

The dispersed phase composition further includes porogen (b3). Porogen does not participate in the polymerization reaction, but is a component that can form pores in the polymerize particles while escaping from the dispersed phase droplet during the suspension polymerization reaction.

In an embodiment of the present application, the porogen(b3) may be a porogen having a difference in Hansen solubility parameters from the epoxy group-containing polymerizable monomer(b1) of 1.30 to 4.70, or may include such a porogen. Specifically, the difference in Hansen solubility parameters between the epoxy group-containing polymerizable monomer (b1) and the porogen may be 1.40 or more, 1.50 or more, 1.60 or more, 1.70 or more, 1.80 or more, 1.90 or more, 2.00 or more, 2.10 or more, 2.20 or more, 2.30 or more, 2.40 or more, 2.50 or more, 2.60 or more, 2.70 or more, 2.80 or more, 2.90 or more, 3.00 or more, 3.10 or more, 3.20 or more, 3.30 or more, 3.40 or more, 3.50 or more, 3.60 or more, 3.70 or more, 3.80 or more, 3.90 or more, or 4.00 or more. And, the upper limit thereof may be, for example, 4.60 or less, 4.50 or less, 4.40 or less, 4.30 or less, 4.20 or less, 4.10 or less, 4.00 or less, 3.90 or less, 3.80 or less, 3.70 or less, 3.60 or less, 3.50 or less, 3.40 or less, 3.30 or less, 3.20 or less, 3.10 or less, or 3.00 or less. As a result of experimental confirmation, if the difference in Hansen solubility parameters is satisfied, it is possible to provide particles with a porous structure that allows the target material to reach the center of the particles while having surface pores large enough to allow the target material to flow into the inside of the particles.

In one exemplary embodiment, a non-aromatic material can be used as the porogen. For example, the porogen may be or include an acetate-based material (porogen). In an embodiment, the porogen component may be at least one selected from the group consisting of butyl acetate, isobutyl acetate, amyl acetate, isoamyl acetate, hexyl acetate, isohexyl acetate, and heptyl acetate. When using an acetate-based porogen that satisfies the values related to the solubility parameters mentioned above, it is advantageous in ensuring that the particles have sufficient purification capacity in affinity chromatography applications. Specifically, when another type of porogen (e.g., aromatic porogen such as toluene or mesitylene) is used instead of the acetate-based porogen mentioned above, it was experimentally confirmed that the deviation regarding the degree of occurrence of cracked particles is large. That is, when particles are prepared as in the present application, the proportion of cracked particles can be greatly reduced (particles having no cracks are stably formed), particles with sufficiently large pores can be provided, which is therefore advantageous in reducing non-specific adsorption.

In an embodiment of the present application, the dispersed phase composition may further include an initiator(b4). The type of initiator is not particularly limited as long as it does not interfere with ensuring the particle characteristics required in the present application. For example, initiators such as an organic peroxide initiator or an azo initiator can be used. Specifically, compounds such as benzoyl peroxide, di-t-amyl peroxide, t-butyl peroxybenzoate, 2,5-dimethyl-2,5 di-(t-butylperoxy)hexane, 2,5-dimethyl-2,5-di-(t-butylperoxy)hexyne-3, di-cumyl peroxide, lauryl peroxide, 2,2'-azobis(2,4-dimethylvaleronitrile), and mixtures thereof can be used, but are not limited thereto.

In an embodiment of the present application, the dispersed phase composition may include 50 parts by weight or more of the polymerizable monomer(b1) based on 100 parts by weight of the crosslinking agent(b2). For example, the dispersed phase composition may include the polymerizable monomer(b1) in the amount of 60 parts by weight or more, 70 parts by weight or more, 80 parts by weight or more, 90 parts by weight or more, 100 parts by weight or more, 110 parts by weight or more, 120 parts by weight or more, 130 parts by weight or more, 140 parts by weight or more, 150 parts by weight or more, 160 parts by weight or more, 170 parts by weight or more, 180 parts by weight or more, 190 parts by weight or more, or 200 parts by weight or more based on 100 parts by weight of the crosslinking agent(b2). The upper limit of the content of the polymerizable monomer(b1) in the dispersed phase composition may be, for example, 400 parts by weight or less, 350 parts by weight or less, 300 parts by weight or less, 250 parts by weight or less, 200 parts by weight or less, 150 parts by weight or less, or 100 parts by weight or less. Further, in the specific embodiment of the present application, the polymerizable monomer(b1) may be included in an amount of 100 parts by weight or more, 150 parts by weight or more, or 200 parts by weight or more based on 100 parts by weight of the crosslinking agent(b2). When the above-mentioned content is satisfied, an emulsion is stably formed, particle breakage during polymerization is greatly suppressed, and an appropriate level of particle strength can be ensured without making the pore size of the final particle excessively large or small. In particular, when the content of components (b1) and (b2) satisfies the above range, the proportion of cracked particles can be reduced (particles having no cracks are stably formed), particles with sufficiently large pores can be provided, which is therefore advantageous in reducing non-specific adsorption.

In an embodiment of the present application, the dispersed phase composition may contain the porogen (b3) in an amount of 45% by weight or more based on 100% by weight of the total content of the polymerizable monomer(b1), the crosslinking agent(b2), and the porogen(b3). For example, based on the total content of 100% by weight, the content of porogen (b3) (which may be an acetate-based porogen by way of example) may be 50% by weight or more, 55% by weight or more, or 60% by weight or more. And, the upper limit of the content may be, for example, 70% by weight or less, 65% by weight or less, 60% by weight or less, or 55% by weight or less. If the above-mentioned content is satisfied, it is advantageous in stably forming an emulsion and ensuring particle characteristics suitable for the intended use (e.g., characteristics of less particle breakage and high spheroidization rate). In particular, when the content of components (b1) and (b2) satisfies the above range, the proportion of cracked particles can be reduced (particles having no cracks are stably formed), and particles with sufficiently large pores can be provided, which is therefore advantageous in reducing non-specific adsorption.

The content of the initiator (b4) is not particularly limited. The initiator may be used in an appropriate amount at a level that does not interfere with ensuring the desired particle characteristics. For example, the dispersed phase composition may contain the initiator in an amount of 0.1 parts by weight or more, specifically 0.2 parts by weight or more, 0.3 parts by weight or more, 0.4 parts by weight or more, 0.5 parts by weight or more, 0.6 parts by weight or more, 0.7 parts by weight or more, 0.8 parts by weight or more, 0.9 parts by weight or more, or 1.0 parts by weight or more with respect to 100 parts by weight of the total weight of the polymerizable monomer and the crosslinking agent. And, the upper limit of the content of the initiator may be, for example, 5.0 parts by weight or less, specifically, 4.5 parts by weight or less, 4.0 parts by weight or less, 3.5 parts by weight or less, 3.0 parts by weight or less, 2.5 parts by weight or less, 2.0 parts by weight or less, 1.5 parts by weight or less, or 1.0 parts by weight or less.

In one exemplary embodiment, the continuous phase composition(A) may include water and a polymeric surfactant.

Although not particularly limited, water may be distilled water or deionized water (DIW).

In one exemplary embodiment, the continuous phase composition may be a mixture of water and a polymeric surfactant. That is, the continuous phase composition may consist of only water and a polymeric surfactant.

In embodiments of the present application, the polymeric surfactant may have a weight average molecular weight($M_W$) in a predetermined range and/or a hydration degree (hydrolyzed degree) in a predetermined range.

In one exemplary embodiment, the weight average molecular weight of the polymeric surfactant may be 60,000 or more. Specifically, the lower limit of the weight average molecular weight may be, for example, 65,000 or more, 70,000 or more, 80,000 or more, 85,000 or more, 90,000 or more, 95,000 or more, 100,000 or more 105,000 or more 110,000 or more 115,000 or more 120,000 or more 125,000 or more 130,000 or more 135,00 0 or more 140,000 or more. And, the upper limit thereof may be 200,000 or less. Specifically, the upper limit of the weight average molecular weight may be, for example, 190,000 or less, 185,000 or less, 180,000 or less, 175,000 or less, 170,000 or less, 165,000 or less, 160,000 or less, 155,000 or less, 150,000 or less, 145,000 or less, 140,000 or less, 135,000 or less, 130,000 or less, 125,000 or less, 120,000 or less, 115,000 or less, or 110,000 or less. The weight average molecular weight can be measured using GPC.

In one exemplary embodiment, the polymeric surfactant may have a hydration degree in the range of 80 to 99%. More specifically, the lower limit of the hydration degree may be, for example, 81% or more, 82% or more, 83% or more, 84% or more, or 85% or more, and the upper limit thereof may be, for example, 98% or less, 97% or less, 96% or less, 95% or less, 94% or less, 93% or less, 92% or less, 91% or less, or 90% or less. For example, polyvinyl alcohol can be used as a surfactant having the above hydration degree. The hydration degree can be measured using 1H-NMR.

Examples of polymeric surfactants that can be used include poly(vinyl alcohol) (PVA) or polyvinylpyrrolidone.

In one exemplary embodiment, in the continuous phase composition, the concentration of the polymeric surfactant may be 1.0% or more. For example, when using a continuous phase composition which is a mixture of water and a polymeric surfactant, the content of the polymeric surfactant may be 1.0% by weight or more based on the total weight (100% by weight) of the continuous phase composition. Specifically, the lower concentration limit of the polymeric surfactant in the continuous phase composition(A) may be 1.5% by weight or more, 2.0% by weight or more, 2.5% by weight or more, 3.0% by weight or more, or 3.5% by weight or more, and the upper limit of the polymeric surfactant concentration may be less than 4.0%, specifically, for example, 3.5% or less, 3.0% or less, 2.5% or less, 2.0% or less, or 1.5% or less. When a polymeric surfactant that satisfies the above concentration is used, uniform and stable droplets can be formed when mixing the continuous phase and the powder phase, which is advantageous in obtaining the shape of the final particle intended by the present application.

In one exemplary embodiment, the weight ratio of the dispersed phase composition to the continuous phase composition (weight ratio of the dispersed phase composition : weight ratio of the continuous phase composition) may be in the range of 1:2 to 1: 15, or in the range of 1:4 to 1: 12, or in the range of 1:6 to 1: 10.

The suspension polymerization can be performed under conditions that do not interfere with ensuring particle characteristics according to the preparation method of the present application. For example, the suspension polymerization reaction may be performed at a temperature of 100°C or less, more specifically at a temperature of 40 to 95°C. Further, during the polymerization reaction at the above temperature, stirring can be performed at a speed of 600 to 1,000 rpm.

In one exemplary embodiment, the method may further include a washing step after completion of the suspension polymerization reaction. By this washing, impurities unrelated to particles which are the suspension polymerization product can be removed. The washing method is not particularly limited, and any known washing method can be used. For example, the washing may be performed by adding the suspension polymerization product to a washing solution containing alcohol (e.g., alcohol containing ethanol) and/or high-temperature (e.g., 40~60°C) water (e.g., distilled water) followed by stirring. Although not particularly limited, this washing may be repeated, for example, two or more times.

In one exemplary embodiment, the method may further include a drying step after the washing. Solvent residues and the like can be removed by drying. The drying method is not particularly limited, and any known drying method can be used. For example, the drying can be performed using an oven or under room temperature conditions. Further, although not particularly limited, the drying may be performed in a vacuum.

In one exemplary embodiment, the method may further include centrifuging, washing, and drying the suspension polymerization product after completion of the suspension polymerization reaction. The details regarding the washing and drying are the same as those described above.

The suspension polymerization product, i.e., particles, prepared according to the above method including suspension polymerization, may have characteristics suitable for protein purification or separation applications.

In one exemplary embodiment, the particles prepared according to the method of the present application may have a size within the range of 30 to 70 $\mu$m. Herein, the particle size means the particle size ($D_{50}$) measured using PSA (particle size analysis) device. And, $D_{50}$ means the particle size corresponding to 50% cumulative volume in the particle size distribution. Specifically, the particles prepared according to the present application may have a lower limit size of 35

μm or more, 40 μm or more, or 45 μm or more, and an upper limit size of 65 μm or less, 60 μm or less, 55 μm or less, 50 μm or less, or 45 μm or less. In the case of exceeding the above particle range, the number of particles that can be packed in the purification column decreases, the content of the ligand attached to the particle surface decreases, and ultimately the purification efficiency may decrease. In addition, when the particle size is smaller than the above particle size, the density of the particles (packed beads) packed in the purification column becomes excessively large, making it difficult for the solution to be purified to escape, which may be therefore lowered in the purification efficiency.

In one exemplary embodiment, the method of the present application can provide a particle group with a low percentage of cracked particles. For example, the proportion of the number of cracked particles may be 20% or less, 15% or less, 10% or less, or 5% or less of the total particles prepared. That is, according to the present application, particles having a stable shape are provided. If the proportion of cracked particles is high, it can be seen that the preparation efficiency is low. Furthermore, when particles are used for protein purification, the purification efficiency may decrease as cracked particles clog the channels through which the solution flows, and the product purity may decrease as cracked particles escape through the column discharge port.

In one exemplary embodiment, the particles may exhibit a high spheroidization rate. For example, the spheroidization rate calculated as the ratio of the particle size ($D_{50}$) to the length (L) of the particles having the longest dimension length (L) among the prepared total particles may be 80% or more, 85% or more, or 90% or more. That is, according to the present application, uniform particles with a high degree of spheroidization are provided. If the spheroidization rate is low, the purification efficiency may be lowered because the particles are not sufficiently packed in the column.

In one exemplary embodiment, the particles may have a pore size of 30 nm or more when the pore size is measured using a mercury intrusion method. The mercury intrusion method is a measurement method that converts the pore size through the amount of mercury introduced by pressure from the surface to the inside of the particles. According to embodiments of the present application, the measured data related to the mercury intrusion method may be represented by a histogram, and the size of the particle pores can be calculated from the measurement of the total intrusion volume at the maximum mercury injection pressure and the ratio of the total pore area (wherein, the size of the particle pores can be considered an average size, and the average can mean an arithmetic mean). If the pores of the particle are too small, only impurities with a small size relative to the target protein will flow into and be adsorbed inside the particle, which may lead to a reduction in purification efficiency due to an increase of non-specific adsorption. For example, the pore size of the particles may be 35 nm or more, 40 nm or more, 45 nm or more, 50 nm or more, 55 nm or more, or 60 nm or more. Further, the upper limit of the average pore size of the particles may be 500 nm or less. When the average pores of the particles are too large, the retention time of impurities in the interparticle voids smaller than the particle pores may increase, and the purification efficiency may decrease due to an increase of non-specific adsorption. Further, due to a decrease in the specific surface area inside the particles, the content of attached ligand may decrease, which may be therefore lowered in the purification efficiency. For example, the pore size of the particles may be 400 nm or less, 300 nm or less, 200 nm or less, or 100 nm or less.

In one exemplary embodiment, the average surface pore size of the particles may be in the range of 110 to 240 nm. In an embodiment of the present application, the average surface pore size is related to the degree of openness of the particle surface, and may mean an (arithmetic) average value calculated after measuring 20 or more pore sizes (wherein, the pore size means the length of the largest dimension in the pore shape) on an SEM image of the particle surface. It is not possible to ascertain the degree of openness of the particle surface only by the pore size of the particle determined by the mercury intrusion method. Considering the flow of the target material or fluid through the particles, the specific surface area of the particles, the mechanical strength of the particles, and the like, the average surface pore sizer may be, for example, 120 nm or more, 130 nm or more, 140 nm or more, 150 nm or more, 160 nm or more, 170 nm or more, 180 nm or more, 190 nm or more, or 200 nm or more, and the upper limit thereof may be, for example, 230 nm or less, 220 nm or less, 210 nm or less, 200 nm or less, 190 nm or less, 180 nm or less, 170 nm or less, 160 nm or less, or 140 nm or less.

In one exemplary embodiment, the porosity of the particles may be in the range of 15 to 40%. Specifically, the porosity of the particles may be, for example, 16% or more, 17% or more, 18% or more, 19% or more, 20% or more, 21% or more, 22% or more, 23% or more, 24% or more, 25% or more, and 39% or less, 38% or less, 37% or less, 36% or less, 35% or less, 34% or less, 33% or less, 32% or less, 31% or less, or 30% or less. If the porosity is smaller than the above-mentioned range, it is difficult to expect high purification efficiency because the specific surface area of the particles is low. If the porosity exceeds the above-mentioned range, the mechanical strength may deteriorate.

In an embodiment of the present application, the method further comprises a predetermined surface treatment step for the suspension polymerization product. Specifically, the method further includes a step of making the surface of the suspension polymerization product hydrophilic through deactivation of epoxy groups.

The particles obtained by the above-mentioned suspension polymerization have an epoxy group derived from an epoxy group-containing polymerizable monomer. In the present application, an epoxy deactivator (e.g., a compound having an epoxy reactive group and a hydrophilic group different therefrom) is reacted with the particles formed by the suspension polymerization to deactivate epoxy groups on the surface of the particles and introduce a hydrophilic group to the surface of the particles. By introduction of the hydrophilic group through epoxy group deactivation, non-specific adsorption caused by hydrophobic bonds between impurity proteins and purified particles can be reduced.

As mentioned above, in an embodiment of the present application, at least 50 parts by weight of the epoxy group-containing polymerizable monomer(b1) is used relative to 100 parts by weight of the crosslinking agent (b2), so that the particles, which are the suspension polymerization product, have a sufficient amount of epoxy groups on their surfaces. Since a sufficient amount of epoxy groups on the particle surface is ensured, the particle surface can be sufficiently hydrophilized due to epoxy deactivation when the epoxy deactivator and the particles react with each other.

The hydrophilization may be performed by reacting an epoxy deactivator having an epoxy reactive group and a hydrophilic group different therefrom with the suspension polymerization product having the epoxy group. For example, the above-mentioned epoxy group deactivation can be achieved by injecting the prepared particles into an epoxy deactivator or a solution containing it. The solvent used at this time is not particularly limited. Further, the reaction for making the surface hydrophilic can be performed, for example, within the range of 30 to 50°C for 10 to 30 hours.

In one exemplary embodiment, the epoxy deactivator may be a compound having an epoxy reactive group and a hydrophilic group different therefrom. The epoxy reactive group may be, for example, a thiol group or an amine group, and the hydrophilic group different from the epoxy reactive group may be, for example, a hydroxy group.

For example, the deactivator may include at least one selected from 1-thioglycerol, ethanolamine, and 2-mercaptoethanol. For example, when 1-thioglycerol is used as a deactivator, deactivation of the epoxy group occurs by an epoxy-thiol reaction, and when ethanolamine is used as a deactivator, deactivation of the epoxy group occurs by an epoxy-amine reaction, wherein the former case is more advantageous in terms of reaction efficiency. Therefore, it may be preferable to use an epoxy deactivator, such as 1-thioglycerol, which contains a thiol group as an epoxy reactive group and a hydroxy group as a hydrophilic group different from the epoxy reactive group.

In an embodiment of the present application, the method comprising: mixing a continuous phase composition(A) and a dispersed phase composition(B) and then subjecting the mixture to a suspension polymerization reaction to prepare a suspension polymerization product including a unit derived from the polymerizable monomer(b1) and the crosslinking agent(b2); and making the surface of the suspension polymerization product hydrophilic through epoxy group deactivation can provide porous particles that exhibit low non-specific adsorption rates. For example, the method can provide the porous particles having a non-specific adsorption rate to BSA (Bovine serum albumin) of 1.5% or less. Specifically, the particles prepared according to the above method can satisfy a non-specific adsorption rate for BSA (Bovine serum albumin) of 1.0% or less, more specifically, 0.5% or less, 0.45% or less, 0.4% or less, 0.35% or less, 0.3% or less, 0.25% or less, 0.2% or less, 0.15% or less, 0.1% or less, 0.05% or less, or 0.01% or less. The non-specific adsorption rate is measured for a mixture containing porous particles, BSA, and water, and is calculated as the ratio of the content (weight) of BSA adsorbed on the porous particles to the content (weight) of the total BSA added to the mixture. According to an embodiment of the present application, the porous particles and the BSA in the mixture for measuring the non-specific adsorption rate may be included in a weight ratio of 1 to 3 porous particles: 1 BSA.

In one exemplary embodiment, the above method can provide particles that satisfy a predetermined hydrophilic group content. Specifically, the method can provide particles that satisfy a hydrophilic group content ($\mu$mol/g) per particle unit weight of 500 $\mu$mol/g or more, specifically, 1000 $\mu$mol/g or more. Wherein, the content of hydrophilic groups per particle unit weight ($\mu$mol/g) can be calculated by multiplying the epoxy group content ($\mu$mol/g) of the suspension polymerization product, which is obtained by suspension polymerization before the surface hydrophilization treatment, by the number of hydrophilic groups in the epoxy deactivator. In addition, the epoxy group content ($\mu$mol/g) of the suspension polymerization product obtained by suspension polymerization can be calculated by titrating particles of a predetermined weight using 0.1M NaOH to calculate the epoxy group content ($\mu$mol), and dividing it by the weight of the particles. More specifically, it can be calculated according to the method described in the experiments described below. For example, the hydrophilic group (e.g., hydroxyl group) content ($\mu$mol/g) of the particles provided according to the above method may be 1500 $\mu$mol/g or more, 2000 $\mu$mol/g or more, 2500 $\mu$mol/g or more, 3000 $\mu$mol/g or more, 3500 $\mu$mol/g or more, 4000 $\mu$mol/g or more, or 4500 $\mu$mol/g or more. And, the upper limit of the hydrophilic group content ($\mu$mol/g) of the particles may be, for example, 5000 $\mu$mol/g or less, specifically 4500 $\mu$mol/g or less, 4000 $\mu$mol/g or less, 3500 $\mu$mol/g or less, or 3000 $\mu$mol/g or less.

By treatment with an epoxy deactivator as described above, particles having a hydrophilic group within the above range can provide excellent purification efficiency. Specifically, in the purification process, adsorption between impurity proteins and purified particles occurs mainly through hydrophobic bonds between the impurity proteins and purified particles. Therefore, as the particles have more hydrophilic groups at an appropriate level, the hydrophobic bonds (non-specific adsorption) can be reduced. This tendency is also confirmed in Examples described below.

In one exemplary embodiment, as a result of the epoxy deactivation and hydrophilic treatment as described above, the method can provide porous particles in which the content of epoxy groups on the surface is adjusted to a predetermined level or less. Specifically, the epoxy group content ($\mu$mol) per unit weight (g) of the epoxy-deactivated particles may be 50 $\mu$mol/g or less. This epoxy group content ($\mu$mol/g) can be calculated by titrating particles of a predetermined weight using 0.1M NaOH to calculate the epoxy group content ($\mu$mol) and dividing it by the weight of the particles. More specifically, it can be calculated according to the method described in the experiments described below. Herein, the epoxy group content ($\mu$mol/g) can be calculated by titrating particles of a predetermined weight using 0.1M NaOH to calculate the epoxy group content ($\mu$mol) and dividing it by the weight of the particles. More specifically, it can be calculated according to the method described in the experiments described later. Specifically, the epoxy content of the epoxy deactivated particles may be, for example, 45 $\mu$mol/g or less, 40 $\mu$mol/g or less, 35 $\mu$mol/g or less, 30 $\mu$mol/g or less, 25 $\mu$mol/g or less, 20 $\mu$mol/g or less, 15 $\mu$mol/g or less, 10 $\mu$mol/g or less, or 5.0 $\mu$mol/g or less. The epoxy group of the particles of the present application is derived from a polymerizable material for particle formation, that is, a polymerizable monomer having an epoxy group. However, assuming that the particles are actually used for purifying the target material, if too many epoxy groups remain on the particle surface, these epoxy groups may react or bind with other materials in addition to the target material (e.g. impurities), which may therefore reduce the purification efficiency. Taking this into consideration, particles with deactivated epoxy groups are used in the present application. In addition, the lower limit of the epoxy group content ($\mu$mol) per unit weight of epoxy deactivated particles may be 1 $\mu$mol/g or more, 5 $\mu$mol/g or more, 10 $\mu$mol/g or more, 15 $\mu$mol/g or more, 20 $\mu$mol/g or more, 25 $\mu$mol/g or more, 30 $\mu$mol/g or more, 35 $\mu$mol/g or more, or 40 $\mu$mol /g or more. For reference, if the epoxy group content on the surface of the particles before deactivation is too small, the surface hydrophilization treatment by epoxy deactivation may not be sufficiently performed. Accordingly, when the particles are used as an actual column resin, it may not be able to bind sufficiently to the material that must be bound to the particles (e.g. rProtein A), and thus, it is necessary to use a sufficient amount of the epoxy group-containing polymerizable monomer as described above.

Although not particularly limited, after the epoxy deactivation and hydrophilization treatments, the particles can be washed and/or dried. The washing and drying can be performed in the same manner as described above.

In one embodiment related to the present application, this application relates to porous particles. The porous particles can be prepared by the method mentioned above and, for example, can be used as a column resin for protein purification or separation.

In one embodiment of the present application, the particles contain a polymerization unit derived from the polymerizable monomer having an epoxy group and the crosslinking agent. The types and characteristics of the polymerizable monomer having an epoxy group and the crosslinking agent are the same as described above.

In one exemplary embodiment, the polymerizable monomer may be a monomer containing a carbon-carbon unsaturated bond and an epoxy group (or glycidyl group). The type of such polymerizable monomers is not particularly limited. For example, the polymerizable monomer may include a (meth)acrylate monomer having an epoxy group, more specifically, glycidyl (meth)acrylate, 4,5-epoxybutyl (meth)acrylate, or 9,10-epoxy stearyl (meth)acrylate. However, the types of polymerizable monomers contained in the dispersed phase composition of the present application as these monomers are not specified.

In one exemplary embodiment, a polyfunctional (meth)acrylate can be used as a crosslinking agent. Specifically, the polyfunctional (meth)acrylates, such as ethylene glycol di(meth)acrylate, trimethylolpropane tri(meth)acrylate, pentaerythritol tri(meth)acrylate, dipentaerythritol hexa(meth)acrylate, dipentaerythritol hexamethacrylate or trimethylpropane trimethacrylate can be used as a crosslinking agent. Alternatively, one or more of the crosslinking agents listed above may be used in combination. However, the crosslinking agent is not limited to the materials listed above.

In addition, the particles of the present application may further include materials derived from the above-mentioned preparation method.

In one exemplary embodiment, the porous particles may have a particle size ($D_{50}$) within the range of 30 to 70 $\mu$m. As the description thereof is the same as those set forth above, further discussions on this matter will not be provided.

In one exemplary embodiment, the porous particles may have a ratio of the number of cracked particles to the total particles of 20% or less. As the description thereof is the same as those set forth above, further discussions on this matter will not be provided.

In one exemplary embodiment, the porous particles can satisfy a spheroidization rate of 80% or more. As the description thereof is the same as those set forth above, further discussions on this matter will not be provided.

In one exemplary embodiment, the porous particles may have a pore size of 30 nm or more. As the description thereof is the same as those set forth above, further discussions on this matter will not be provided.

In one exemplary embodiment, the porous particle may have a surface pore size ranging from 110 to 240 nm. As the description thereof is the same as those set forth above, further discussions on this matter will not be provided.

In one exemplary embodiment, the porous particles may have a porosity ranging from 15 to 40%. As the description thereof is the same as those set forth above, further discussions on this matter will not be provided.

In one exemplary embodiment, the particles may be hydrophilically-treated, i.e., surface-treated with an epoxy deactivator having an epoxy reactive group and a hydrophilic group different from the epoxy reactive group.

Epoxy deactivation and hydrophilic treatment for particles may mean that the particles have been surface-treated with an epoxy deactivator having an epoxy reactive group and a hydrophilic group different from the epoxy reactive group. For details of such surface treatment, refer to those set forth above.

In one exemplary embodiment, the porous particles may be particles that satisfy a non-specific adsorption rate for BSA (Bovine serum albumin) of 1.5% or less. As the description thereof is the same as those set forth above, further discussions on this matter will not be provided.

In one exemplary embodiment, as a result of the epoxy deactivation and hydrophilic treatment as mentioned above, the porous particles can satisfy a predetermined hydrophilic group content. Specifically, the porous particle can satisfy a hydrophilic group content ($\mu$mol/g) per unit weight of the epoxy-deactivated particle of 500 $\mu$mol/g or more, specifically, 1000 $\mu$mol/g or more. As the description thereof is the same as those set forth above, further discussions on this matter will not be provided.

In one exemplary embodiment, as a result of the epoxy deactivation and hydrophilic treatment as mentioned above, the porous particles may satisfy a predetermined epoxy group content. Specifically, the porous particles may have an epoxy group content ($\mu$mol) per unit weight (g) of the epoxy-deactivated particle of 50 $\mu$mol/g or less. As the description thereof is the same as those set forth above, further discussions on this matter will not be provided.

### [Advantageous Effects]

According to an embodiment of the present application, when used for protein purification particles (column resin), particles capable of improving non-specific adsorption of impurities to the particles are provided. Accordingly, the binding capacity of the antibody protein to the particles may be sufficient, and high purity of the final product can be ensured.

### [BRIEF DESCRIPTION OF THE DRAWINGS]

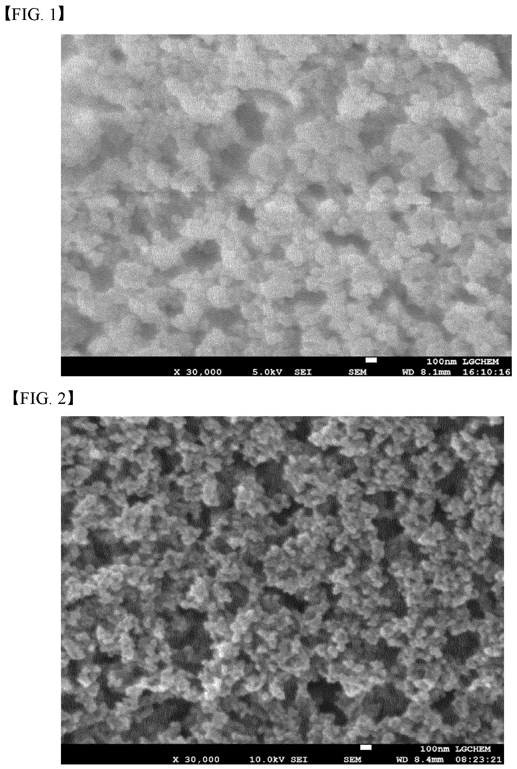

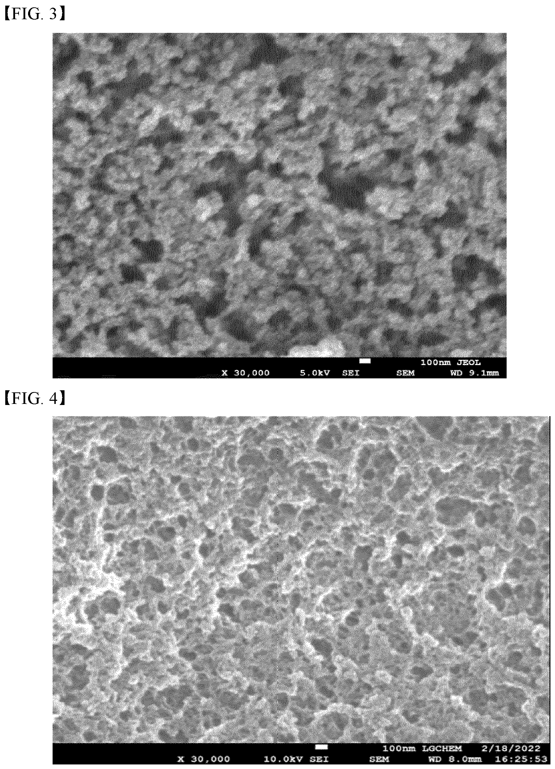

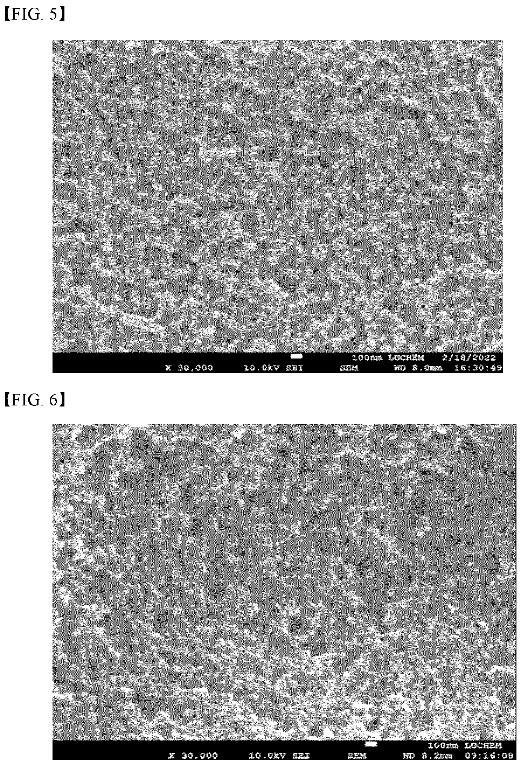

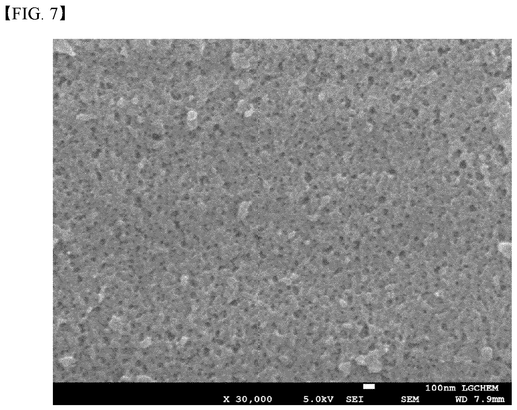

FIGS. 1 to 7 are images of the surface of particles (base resin) prepared in Examples and Comparative Examples. Specifically, FIGS. 1 to 3 are images of the surface of the particles (base resin) of Examples 1 to 3, respectively, taken using SEM device, and FIGS. 4 to 7 are images of the surface of the particles (base resin) of Comparative Examples 5 to 7 and Comparative Example 11, respectively, taken using SEM device. In each figure, the white bar represents the size of 100 nm, and the black parts observed in the particles are pores.

Comparing the drawings of Examples and Comparative Examples, the black parts, i.e., pores, are more visible in the images of Examples than in the images of Comparative Examples. This means that the pores or surface pores of the particles of Examples are larger and more numerous than those of the particles of Comparative Examples. This means that in the case of Comparative Examples (e.g. Comparative Examples 5~7), the pore size measured by MIP of the particles is at the level of 10~11 nm, and in the case of Examples (e.g., Examples 1~2), the pore size measured by MIP of the particles is consistent with the experimental results below (see Tables 5~6), which state that the pore size is at the level of 35~40 nm. Further, looking at the experimental results in Tables 5~6, it is confirmed that Examples (120~160 nm level) and Comparative Examples (level of 105 nm or less. Particularly, Comparative Example 11 is confirmed to be 45 nm) show a clear difference in average surface pore size.

### [DETAILED DESCRIPTION OF THE EMBODIMENTS]

Hereinafter, actions and effects of the invention will be described in more detail with reference to specific examples. However, this is for illustrate purposes only, and are not intended to limit the scope of the invention in any way.

### Example and Comparative Example

The particle-forming components, their contents, and process conditions are as listed in Tables below, and the conditions under which each particle was prepared in Examples and Comparative Examples are as follows. If there is no specific explanation regarding temperature, the temperature at which the preparation process is performed (or each preparation step) or the temperature at which the characteristics possessed by the prepared particles are calculated or measured is room temperature (temperature without reduced or heated temperature, refers to a temperature within the range of about 15 to 30°C).

### Preparation of dispersed phase

After stirring a polymerizable monomer (M), a crosslinking agent (C), and porogen (P), BPO, which is a thermal initiator (about 1.25 parts by weight relative to 100 parts by weight of the total mass of the polymerizable monomer and the crosslinking agent) was further added, and stirred at room temperature for about 5 minutes to prepare a dispersed phase.

* GMA: glycidyl metahacrylate
* EGDMA: ethylene glycol dimetahcrylate

* TMPTMA: trimethylolpropane trimethacrylate

Preparation of continuous phase

PVA (weight average molecular weight(Mw) of 85,000 to 124,000 and hydrolysis rate of 87 to 89%) was dissolved in distilled water at a concentration of 2 wt.% to prepare a continuous phase.

Preparation of particles (base resin) by suspension polymerization

720 g of the continuous phase was added to a reactor, and 90 g of the dispersed phase was added, and the mixture was stirred at about 800 rpm at room temperature until a uniform dispersion liquid was formed. Subsequently, the reactor temperature was raised, and polymerization was performed at a temperature of about 80°C, about 800 rpm, and nitrogen purging conditions for about 6 hours.

Obtaining particles

The polymerized particles were recovered, washed twice with distilled water at 50°C, and five times with ethanol. Subsequently, it was dried in an oven at about 80°C to obtain particles.

[Table 1]

| Preparation conditions of particles (base resin) of Examples 1~4 | | | | | |
|---|---|---|---|---|---|
| | | Example 1 | Example 2 | Example 3 | Example 4 |
| Particle component | Polymerizable monomer (M) | GMA | GMA | GMA | GMA |
| | Crosslinking agent (C) | EGDMA | EGDMA (C1)+ TMPTMA(C2) | EGDMA (C1)+ TMPTMA(C2) | EGDMA |
| | Porogen(P) | Amyl acetate | Amyl acetate | Amyl acetate | N-butyl acetate |
| | Weight ratio | 2(M) : 1(C) : 3(P) | 2(M): 0.5(C1) : 0.5(C2): 3(P) | 1.5(M) : 0.6(C1) : 0.4(C2) : 3(P) | 2(M): 1(C) : 3(P) |
| | Difference in solubility parameters between polymerizable monomer and porogen | 4.34 | 4.34 | 4.34 | 3.95 |

[Table 2]

| Preparation conditions of particles (base resin) of Comparative Examples 1~4 | | | | | |
|---|---|---|---|---|---|
| | | Comparative Example 1 | Comparative Example 2 | Comparative Example 3 | Comparative Example 4 |
| Particle component | Polymerizable monomer(M) | GMA | GMA | GMA | GMA |
| | Crosslinking agent (C) | EGDMA | EGDMA | EGDMA(C1) + TMPTMA(C2) | EGDMA(C1) + TMPTA(C2) |
| | Porogen(P) | Toluene | Mesitylene | Mesitylene | Toluene |
| | Weight ratio | 1(M) : 3(C) : 3(P) | 1(M) : 3(C) : 3(P) | 1(M) : 1.5(C1) : 1.5(C2) : 4(P) | 1(M): 1.5(C1) : 1.5(C2) : 6(P) |
| | Difference in solubility parameters between polymerizable monomer and porogen | 8.21 | 9.73 | 9.73 | 8.21 |

[Table 3]

| Preparation conditions of particles (base resin) of Comparative Examples 5~8 | | | | | |
|---|---|---|---|---|---|
| | | Comparativ e Example 5 | Comparativ e Example 6 | Comparative Example 7 | Comparative Example 8 |
| Particle componen t | Polymerizabl e monomer (M) | GMA | GMA | GMA | GMA |
| | Crosslinking agent (C) | EGDMA | EGDMA | EGDMA(C1) + TMPTA(C2) | EGDMA(C1) + Gly-cerin dimethacryla-te(C2 ) |
| | Porogen (P) | Amyl acetate | Hexyl acetate | Hexyl acetate | Mesitylene |
| | Weight ratio | 1(M) : 3(C) : 3(P) | 1(M) : 3(C) : 3(P) | 1(M) : 1.5(C1) : 1.5(C2) : 4(P) | 1(M) : 1.5(C1) : 1.5(C2) : 3(P) |
| | Difference in solubility parameters between polymerizabl e monomer and porogen | 4.34 | 4.74 | 4.74 | 9.73 |

[Table 4]

| Preparation conditions of particles (base resin) of Comparative Examples 9~12 | | | | | |
|---|---|---|---|---|---|
| | | Comparative Example 9 | Comparative Example 10 | Comparative Example 11 | Comparative Example 12 |
| Particle component | Polymerizabl e monomer(M) | GMA | GMA | GMA | GMA |
| | Crosslinking agent (C) | Glycerin dimethacrylate(C ) | EGDMA (C1) + Glycerin dimethacrylat e (C2) | EGDMA | EGDMA |
| | Porogen(P) | Mesitylene | Amyl acetate | Dimethyl adi-pate | Glycerol dia-cetate |
| | Weight ratio | 1(M) : 3(C) : P(3) | 1(M) : 1.5(C1) : 1.5(C2) : 3(P) | 2(M) : 1(C) : P(3) | 2(M) : 1(C) : P(3) |
| | Difference in solubility parameters between polymerizable monomer and porogen | 9.73 | 4.34 | 1.28 | 6.88 |

## Evaluation 1: Characteristic evaluation of base resin

The particles prepared in Examples and Comparative Examples were evaluated according to the following contents.

### 1. Particle (base resin) size

The prepared particles were dried, the dried particles were dispersed in ethanol at a level of about 10%, and then the particle size (D50) was measured using a PSA device. At this time, D50 means the particle size corresponding to 50% cumulative volume in the particle size distribution.

### 2. Spheroidization rate (%) of particles (base resin)

The spheroidization rate was determined by confirming the length (L) of the longest dimension among the prepared particles through SEM, and calculating the percentage ratio (D50/L) of the particle size (D50) measured in the item 1 to the length (L).

### 3. Crack rate (%) of particles (base resin)

The particle crack rate was determined by confirming the number of particles that have defects on the surface or are cracked without maintaining their spherical shape among the prepared particles through SEM, and calculating the percentage ratio of the number of particles maintaining a spherical shape relative to the total particles.

### 4. Shape of particle (base resin) pores

The pores formed on the surface and inside of the dried particles were observed using a SEM (JEOL JSM7610F-Plus) device.

### 5. Pore size of particles (base resin) and porosity of particles

The pore size of the particles was measured using Mercury intrusion porosimetry (MIP) (Autopore V9600, micromeritics).

### 6. Surface pore size

20 or more pore sizes (wherein the pore size means the length of the largest dimension in the pore shape) were determined on a SEM image of the particle surface, and the arithmetic mean value was calculated. By the calculated surface pore size, the degree of openness of the particle surface (which cannot be determined by MIP) can be indirectly confirmed.

[Table 5]

| Characteristics of particles (base resin) of Examples | | | | | | | |
|---|---|---|---|---|---|---|---|
| | Particle size (D50, μm) | Particle spheroidization rate (%) | Particle crack rate (%) | Pore shape image | Particle pore size by MIP (nm) | Porosity of particles (%) | Average surface pore(nm) |
| Example 1 | 49.8 | > 90 | < 5 | See FIG. 1 | 40.1 | 20.5 | 146 |
| Example 2 | 51.6 | > 90 | < 5 | See FIG. 2 | 38.4 | 20.3 | 159 |
| Example 3 | 50.8 | > 90 | < 10 | See FIG. 3 | 35.4 | 21.1 | 131 |
| Example 4 | 48.8 | > 90 | < 10 | - | 35.1 | 20.8 | 123 |

[Table 6]

| Characteristics of particles (base resin) of Comparative Examples | | | | | | | |
|---|---|---|---|---|---|---|---|
| | Particle size (D50, μm) | Particle spheroidization rate (%) | Particle crack rate(%) | Pore shape image | Particle pore size by MIP (nm) | Porosity of particles (%) | Average surface pore(nm) |
| Comparative Example 1 | 61.45 | > 90 | < 20 | - | 17.8 | 17.2 | 81 |
| Comparative Example 2 | 41.9 | > 90 | < 5 | - | 24.6 | 18.0 | 100 |
| Comparative Example 3 | 44.6 | > 90 | < 5 | - | 27.7 | 20.2 | 105 |
| Comparative Example 4 | 46.5 | > 90 | < 5 | - | 21.8 | 21.4 | 89 |
| Comparative Example 5 | 54.1 | > 90 | < 5 | See FIG. 4 | 11.1 | 12.4 | 82 |

(continued)

| Characteristics of particles (base resin) of Comparative Examples | | | | | | | |
|---|---|---|---|---|---|---|---|
| | Particle size (D50, $\mu$m) | Particle spheroidization rate (%) | Particle crack rate(%) | Pore shape image | Particle pore size by MIP (nm) | Porosity of particles (%) | Average surface pore(nm) |
| Comparative Example 6 | 53.7 | > 90 | < 5 | See FIG. 5 | 11.7 | 12.8 | 79 |
| Comparative Example 7 | 49.2 | > 90 | < 5 | See FIG. 6 | 10.8 | 13.9 | 62 |
| Comparative Example 8 | - | - | > 90 | - | - | - | - |
| Comparative Example 9 | - | - | > 90 | - | - | - | - |
| Comparative Example 10 | 44.2 | > 90 | < 10 | - | 9.8 | 9.3 | 99 |
| Comparative Example 11 | 45.1 | > 90 | < 5 | See FIG. 7 | 7.8 | 8.1 | 45 |
| Comparative Example 12 | - | - | > 90 | - | - | - | - |
| * In the case of Comparative Examples 8~9 and 12, the crack rate of the particles was high, and thus, evaluation regarding particle characteristics could not be conducted. | | | | | | | |

**Evaluation 2: Evaluation of non-specific adsorption rate**

The particles prepared in Examples and Comparative Examples were processed according to the following contents, and the related contents were evaluated.

**1. Epoxy content, SBC and non-specific adsorption rate of particles (base resin)**

(1) Analysis of epoxy content ($\mu$mol/g) of particles (base resin)

1) 0.1 g of particles (DI water was used for a control) were mixed with 1 mL HCl/acetone solution (1/40(v/v)) and sonicated for 4 minutes (UCP-02 from Jeiotech).
2) An indicator solution (0.1% cresol red and 0.1% thymol blue were mixed in a 1:3 volume ratio and adjusted to pH 7.0) was added to the mixed solution in the item 1).
3) Titration was performed with 0.1M NaOH until the color of the solution becomes purple-blue.
4) The epoxy content was calculated using the following Equation.

$$[\text{Equation }] \text{ Epoxy content (mmol)} = [V_0(ml) - V(ml)] \times C_{NaOH} \text{ (mol/L)}$$

in the above Equation,

V0: Amount of NaOH added to control sample using DI water instead of particles (mL)
V: Amount of NaOH added to the experimental group sample containing epoxy (mL)
CNaOH: NaOH concentration used for titration

5) The calculated epoxy content was divided by the weight of the particles (0.1g was used in the item 1) beforehand), and the epoxy content per weight of particle (base resin) was confirmed.

(2) SBC (Static binding capacity, mg/mL) was measured (Reference: Ind. Eng. Chem. Res. 2013, 52, 26, 8800-8811, Biochemical Engineering Journal 145 (2019) 53-61)

1) 50 μL of a base resin slurry was prepared, and the antibody solution (IgG from human serum, 5 mg/mL PBS, pH 7.4) was thawed, and then 1x PBS was added to prepare the antibody solution at 3 mg/mL.

2) After mixing the resin slurry and the antibody solution, the IgG was bounded to the resin using a mixer at room temperature for 1 hour.

3) After precipitating the resin, the supernatant (or stored as needed) was removed, and washed by injecting 5 mL of 1x PBS.

4) After precipitating the resin, the washing solution (or stored as needed) was removed, and 500 μL of 100 mM glycine-HCl (pH 2.5) eluate was injected to elute the IgG.

5) After precipitating the resin, the eluate was collected and stored at 4°C.

6) The IgG concentration was measured in the eluate at 280 nm using UV-vis (8453 UV-visible spectrophotometer from Agilent). After IgG binding, the IgG content was measured in each of the supernatant, washing solution, and eluate, and the IgG content in each solution was calculated using the IgG calibration curve.

(3) Non-specific adsorption rate (%) when measuring SBC

Based on 100% of the total IgG content (weight) of the supernatant, washing liquid, and eluate confirmed in the item (2)-6) above, the content (%) of IgG confirmed in the washing liquid was substituted into the non-specific adsorption rate (%) when measuring SBC.

**2. Epoxy content (μmol/g), hydrophilic functional group content (μmol/g), and non-specific adsorption rate (%) after epoxy deactivation on particles (base resin).**

(1) Epoxy deactivation

1) The base resin was injected into 1-thioglycerol solution (0.5 M 1-thioglycerol + 0.1 M sodium sulfate + 0.1 M EDTA (pH8.5)), and allowed to react at 37°C for 17 hours. For Comparative Example 1, ethanol amine was used instead of 1-thiolglycerol.

2) The particles was precipitated using a method such as centrifugation, and then the supernatant was removed. Washed 3 times with DIW.

(2) Epoxy content after epoxy deactivation (μmol/g)
Epoxy deactivated particles, that is, particles surface-treated with 1-thioglycerol, were prepared by drying at room temperature, and the epoxy content of particles surface-treated with 1-thioglycerol was measured in the same manner as described above.

(3) Content of hydrophilic functional group (μmol/g)
The hydrophilic functional group content of the particles surface-treated with 1-thioglycerol was measured as shown in <Equation> below.

Hydrophilic functional group content after 1-thioglycerol surface treatment = (Epoxy content of particles before 1-thioglycerol surface treatment)×Number of hydroxyl functional groups in 1- thio-       <Equation> glycerol

(4) Non-specific adsorption rate (%)

The epoxy deactivated particles were dried. Then, the particles were mixed with a 10 mg/mL solution of BSA (sigma A9418) dissolved in DIW at a concentration of 31.3 mg/mL. Then, the mixed solution was stirred at room temperature for 18 hours, washed 5 times in DIW, and dried at room temperature. The non-specific adsorption rate was calculated through the ratio of the content of BSA adsorbed to the particles relative to the total BSA content added during stirring at room temperature. At this time, the content of BSA adsorbed on the particles was analyzed using Pierce™ BCA Protein Assay Kit (Thermo Fischer).

[Table 7]

| Evaluation after epoxy deactivation of particles (base resin) of Examples | | | | | | | |
|---|---|---|---|---|---|---|---|
| | Base resin | | | Base resin treated by epoxy deactivation | | | |
| | Epoxy content ($\mu$mol/g ) | SBC (mg/mL) | Non-specific adsorption rate during SBC measuremen t (%) | Epoxy deactivation Agent | Epoxy content after treatmen t ($\mu$mol/g) | Hydrophili c functional group content after treatment ($\mu$mol/g) | Non-specific adsorptio n rate under condition of BSA 10 mg/mL after treatment (%) |
| Exampl e 1 | 1891 | 0.56 | 4.7 | 1-thioglycero l | < 50 | 3682 | 0.068 |
| Exampl e 2 | 1752 | 0.59 | 4.7 | 1-thioglycero l | < 50 | 3504 | 0.43 |
| Exampl e 3 | 1300 | 0.60 | 5.2 | 1-thioglycero l | < 50 | 2600 | 0.71 |
| Exampl e 4 | 1418 | 0.59 | 4.8 | 1-thioglycero l | < 50 | 2836 | 0.66 |

[Table 8]

| Evaluation after epoxy deactivation of particles (base resin) of Comparative Examples | | | | | | | |
|---|---|---|---|---|---|---|---|
| | Base resin | | | Base resin treated by epoxy deactivation | | | |
| | Epoxy content ($\mu$mol/g) | SBC (mg/mL ) | Non-specific adsorption rate during SBC measureme nt (%) | Epoxy deactivatio n Agent | Epoxy content after treatme nt ($\mu$mol/g ) | Hydrophil ic functional group content after treatment ($\mu$mol/g) | Non-specific adsorptio n rate under condition of BSA 10 mg/mL after treatment (%) |
| Comparati ve Example 1 | 695 | 0.9 | 6.2 | Ethanol amine | 597 | 98 | 4.4 |
| Comparati ve Example 2 | 762 | 0.73 | 7.8 | - | - | - | - |
| Comparati ve Example 3 | 554 | 1.49 | 6.2 | 1-thiolglycer ol | < 50 | 1008 | 8.75 |
| Comparati ve Example 4 | 200 | - | - | 1-thiolglycer ol | < 50 | 400 | 9.81 |
| Comparati ve Example 5 | 503 | - | - | 1-thiolglycer ol | < 50 | 1006 | 6.98 |
| Comparati ve Example 6 | 680 | - | - | 1-thiolglycer ol | < 50 | 1360 | 7.11 |

(continued)

| Evaluation after epoxy deactivation of particles (base resin) of Comparative Examples | | | | | | | |
|---|---|---|---|---|---|---|---|
| | Base resin | | | Base resin treated by epoxy deactivation | | | |
| | Epoxy content ($\mu$mol/g) | SBC (mg/mL ) | Non-specific adsorption rate during SBC measurement (%) | Epoxy deactivatio n Agent | Epoxy content after treatme nt ($\mu$mol/g ) | Hydrophil ic functional group content after treatment ($\mu$mol/g) | Non-specific adsorptio n rate under condition of BSA 10 mg/mL after treatment (%) |
| Comparati ve Exam-ple 7 | 596 | - | - | 1-thiolglycer ol | < 50 | 1192 | 7.29 |
| Comparati ve Example 8 | Impossibl e to measure due to particle cracks | Same as the left | Same as the left | Same as the left | Same as the left | Same as the left | Same as the left |
| Comparati ve Example 9 | Impossibl e to measure due to particle cracks | Same as the left | Same as the left | Same as the left | Same as the left | Same as the left | Same as the left |
| Comparati ve Example 10 | 987 | 0.69 | 7.1 | 1-thiolglycer ol | < 50 | 1974 | 1.55 |
| Comparati ve Example 11 | 2081 | 0.61 | 5.5 | 1-thiolglycer ol | < 50 | 4162 | 1.13 |
| Comparati ve Example 12 | Impossibl e to measure due to particle cracks | Same as the left | Same as the left | Same as the left | Same as the left | Same as the left | Same as the left |

※ In the case of Comparative Example 2, since the non-specific adsorption rate of the base resin itself was confirmed as a high value, subsequent repetitive non-specific adsorption rate tests (i.e., related experiments after epoxy deactivation treatment) were not conducted.

※ In Comparative Example 4, which had a significantly low epoxy content in the base resin itself, a non-specific adsorption rate test for the base resin was not conducted. In the same sense, a non-specific adsorption rate test was not conducted on the base resin of Comparative Examples 5~7.

**Claims**

1. A method for preparing porous particles, the method comprising:

mixing a continuous phase composition(A); and a dispersed phase composition (B) including an epoxy group-containing polymerizable monomer (b1), a crosslinking agent(b2), and a porogen(b3) and then subjecting the mixture to a suspension polymerization reaction to prepare a suspension polymerization product including a unit derived from the polymerizable monomer(b1) and the crosslinking agent(b2),
wherein the dispersed phase composition includes 50 parts by weight or more of the epoxy group-containing polymerizable monomer(b1) based on 100 parts by weight of the crosslinking agent(b2), and
wherein the porogen(b3) includes a porogen in which a difference in Hansen solubility parameters from the epoxy group-containing polymerizable monomer (b1) satisfies the range of 1.30 to 4.70.

2. The method for preparing porous particles according to claim 1, wherein:
the dispersed phase composition includes 150 parts by weight or more of the epoxy group-containing polymerizable monomer(b1) based on 100 parts by weight of the crosslinking agent(b2).

3. The method for preparing porous particles according to claim 1, wherein:
the porogen(b3) includes a porogen in which the difference in Hansen solubility parameters from the epoxy group-containing polymerizable monomer(b1) is 2.50 to 4.50.

4. The method for preparing porous particles according to claim 1, wherein:
the porogen(b3) comprises an acetate-based porogen.

5. The method for preparing porous particles according to claim 1, wherein:
the dispersed phase composition comprises 45% by weight or more of the porogen(b3), based on 100% by weight of the total content of the epoxy group-containing polymerizable monomer(b 1), the crosslinking agent(b2) and the porogen(b3).

6. The method for preparing porous particles according to claim 1, wherein:
the crosslinking agent(b2) comprises a crosslinking agent that does not contain a hydroxyl group(-OH), or is a crosslinking agent that does not contain a hydroxyl group(-OH).

7. The method for preparing porous particles according to claim 1, wherein:
the continuous phase composition comprises water and a polymeric surfactant.

8. The method for preparing porous particles according to claim 1, wherein:

the suspension polymerization product has a particle shape, and
the particles have a $D_{50}$ size in the range of 30 to 70 $\mu$m,
(wherein, $D_{50}$ means the particle size corresponding to 50% cumulative volume in the particle size distribution).

9. The method for preparing porous particles according to claim 1, wherein:

the suspension polymerization product has a particle shape, and
the particles have a pore size in the range of 30 to 500 nm, as measured by the mercury intrusion method, and a particle average surface pore size in the range of 110 to 240 nm, which is the arithmetic average of the sizes of 20 or more pores observed on the particle surface.

10. The method for preparing porous particles according to claim 1, wherein:

the suspension polymerization product has a particle shape, and
the particles have a porosity in the range of 15 to 40%.

11. The method for preparing porous particles according to claim 1,
further comprising making the surface of the suspension polymerization product hydrophilic through deactivation of the epoxy groups.

12. The method for preparing porous particles according to claim 11, wherein:

the suspension polymerization product has an epoxy group derived from the epoxy group-containing polymerizable monomer (b1), and
an epoxy deactivator having an epoxy reactive group and a hydrophilic group different therefrom is reacted with the suspension polymerization product having an epoxy group to make the surface of the suspension polymerization product hydrophilic.

13. The method for preparing porous particles according to claim 12, wherein:

the epoxy deactivator comprises at least one of a thiol group and an amine group as the epoxy reactive group, and comprises a hydroxy group as a hydrophilic group different from the epoxy reactive group.

14. Porous particles comprising a polymerization unit derived from a polymerizable monomer having an epoxy group and a crosslinking agent,
    wherein the particles satisfy the following features:

    a particle size ($D_{50}$) in the range of 30 to 70 $\mu$m,
    a pore size in the range of 30 to 500 nm, as measured by the mercury intrusion method,
    a particle average surface pore size in the range of 110 to 240 nm, which is the arithmetic average of the sizes of 20 or more pores observed on the particle surface, and
    a porosity in the range of 15 to 40%.

15. The porous particles according to claim 14, wherein:
    the porous particles are surface-treated with an epoxy deactivator having an epoxy reactive group and a hydrophilic group different from the epoxy reactive group.

【FIG. 1】

【FIG. 2】

【FIG. 3】

【FIG. 4】

【FIG. 5】

【FIG. 6】

【FIG. 7】

## INTERNATIONAL SEARCH REPORT

| International application No. |
| --- |
| **PCT/KR2023/021323** |

**A.  CLASSIFICATION OF SUBJECT MATTER**

**B01J 20/26**(2006.01)i; **B01J 20/28**(2006.01)i; **B01J 20/30**(2006.01)i; **C07K 1/22**(2006.01)i

According to International Patent Classification (IPC) or to both national classification and IPC

**B.  FIELDS SEARCHED**

Minimum documentation searched (classification system followed by classification symbols)

B01J 20/26(2006.01); B01J 20/285(2006.01); B01J 41/04(2006.01); B01J 41/14(2006.01); C08G 59/62(2006.01); C08J 9/00(2006.01); C08K 3/10(2006.01); C12Q 1/10(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 다공성 입자(porous particle), 연속상(continuous phase), 분산상(dispersed phase), 에폭시기 함유 단량체(monomer containing epoxy group), 가교제(crosslinking agent), 포로겐(porogen), 현탁 중합 (suspension polymerization)

**C.  DOCUMENTS CONSIDERED TO BE RELEVANT**

| Category* | Citation of document, with indication, where appropriate, of the relevant passages | Relevant to claim No. |
| --- | --- | --- |
| X | US 2021-0214514 A1 (EMAUS KYOTO, INC.) 15 July 2021 (2021-07-15)<br>See paragraphs [0096], [0101], [0129], [0130], [0133], [0145], [0185], [0188], [0199] and [0227]; and claims 17 and 18. | 1-15 |
| A | US 2018-0161758 A1 (MITSUBISHI CHEMICAL CORPORATION et al.) 14 June 2018 (2018-06-14)<br>See entire document. | 1-15 |
| A | KR 10-2019-0138780 A (IXOM OPERATIONS PTY LTD.) 16 December 2019 (2019-12-16)<br>See entire document. | 1-15 |
| A | US 2017-0157591 A1 (3M INNOVATIVE PROPERTIES COMPANY) 08 June 2017 (2017-06-08)<br>See entire document. | 1-15 |
| A | KR 10-2015-0052101 A (ORICA AUSTRALIA PTY. LTD.) 13 May 2015 (2015-05-13)<br>See entire document. | 1-15 |

☐ Further documents are listed in the continuation of Box C.  ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **01 April 2024** | **01 April 2024** |

| Name and mailing address of the ISA/KR | Authorized officer |
| --- | --- |
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

<table>
<tr><td colspan="2" align="center"><b>INTERNATIONAL SEARCH REPORT</b><br>Information on patent family members</td><td colspan="2">International application No.<br><br><b>PCT/KR2023/021323</b></td></tr>
</table>

| Patent document cited in search report | Publication date (day/month/year) | Patent family member(s) | Publication date (day/month/year) |
|---|---|---|---|
| US 2021-0214514 A1 | 15 July 2021 | CN 112218913 A | 12 January 2021 |
| | | CN 112218913 B | 27 October 2023 |
| | | EP 3789444 A1 | 10 March 2021 |
| | | EP 3789444 A4 | 02 March 2022 |
| | | JP 7132659 B2 | 07 September 2022 |
| | | US 11613618 B2 | 28 March 2023 |
| | | WO 2020-179642 A1 | 10 September 2020 |
| US 2018-0161758 A1 | 14 June 2018 | EP 3335789 A1 | 20 June 2018 |
| | | EP 3335789 A4 | 18 July 2018 |
| | | JP 2017-037070 A | 16 February 2017 |
| | | US 10773241 B2 | 15 September 2020 |
| | | WO 2017-026453 A1 | 16 February 2017 |
| KR 10-2019-0138780 A | 16 December 2019 | CN 110505914 A | 26 November 2019 |
| | | CN 110505914 B | 04 October 2022 |
| | | EP 3579965 A1 | 18 December 2019 |
| | | EP 3579965 A4 | 16 December 2020 |
| | | EP 3579965 B1 | 05 April 2023 |
| | | JP 2020-506993 A | 05 March 2020 |
| | | JP 7149949 B2 | 07 October 2022 |
| | | KR 10-2607919 B1 | 30 November 2023 |
| | | US 2020-0030786 A1 | 30 January 2020 |
| | | WO 2018-145152 A1 | 16 August 2018 |
| US 2017-0157591 A1 | 08 June 2017 | CN 105828930 A | 03 August 2016 |
| | | CN 105828930 B | 05 July 2019 |
| | | EP 3083036 A1 | 26 October 2016 |
| | | EP 3083036 B1 | 18 March 2020 |
| | | JP 2017-503881 A | 02 February 2017 |
| | | US 10413881 B2 | 17 September 2019 |
| | | WO 2015-095100 A1 | 25 June 2015 |
| KR 10-2015-0052101 A | 13 May 2015 | CN 104684980 A | 03 June 2015 |
| | | CN 104684980 B | 29 March 2017 |
| | | EP 2890734 A1 | 08 July 2015 |
| | | EP 2890734 A4 | 13 April 2016 |
| | | EP 2890734 B1 | 28 February 2018 |
| | | JP 2015-532671 A | 12 November 2015 |
| | | JP 6411347 B2 | 24 October 2018 |
| | | KR 10-2033492 B1 | 17 October 2019 |
| | | US 10173197 B2 | 08 January 2019 |
| | | US 2015-0218015 A1 | 06 August 2015 |
| | | WO 2014-032092 A1 | 06 March 2014 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 1020230013569

- KR 1020230061331

**Non-patent literature cited in the description**

- *Ind. Eng. Chem. Res.,* 2013, vol. 52 (26), 8800-8811

- *Biochemical Engineering Journal*, 2019, vol. 145, 53-61